# EUROPEAN PATENT APPLICATION

(11) **EP 2 612 665 A1**
(43) Date of publication of application: **10.07.2013**
(21) Application number: 12000077.3
(22) Date of filing: 09.01.2012
(51) Int. Cl.: A61K 31/192, A61K 31/573, A61K 9/06, A61K 47/02, A61K 9/00, A61P 17/06

(54) **Topical pharmaceutical compositions comprising bexarotene and a corticosteroide**

(71) Applicant: Almirall S.A., 08022 Barcelona (ES)
(72) Inventor: Willers, Christoph, 21465 Reinbek (DE); Trommer, Hagen, 21465 Reinbek (DE); Mallwitz, Henning, 21465 Reinbek (DE); Fielhauer, Sabine, 21465 Reinbek (DE); Evers, Fritjof, 21465 Reinbek (DE)
(74) Representative: polypatent

(57) **Abstract**

Topical pharmaceutical compositions are described comprising:
a) bexarotene,
b) a corticosteroid, and
c) a carrier or vehicle.

Said compositions are useful for the treatment of skin disorders.

## Description

### FIELD OF THE INVENTION

The present invention relates to topical pharmaceutical compositions comprising bexarotene and corticosteroids. The combination of these two active ingredients produces a significant anti-psoriatic effect and an improved tolerability. The compositions provided are stable and can be easily applied over large surface areas of the skin. The invention further relates to a process for the preparation of the compositions and to methods of treatment by administering them.

### BACKGROUND OF THE INVENTION

Psoriasis is a disease characterised by epidermal proliferation showing premature keratinisation, elongation, dilatation and increased permeability of the superficial capillaries In the dermis. There is an associated immune activation with signs of inflammation, Clinically active lesions are red, raised areas with superficial scale that detaches easily producing a silvery appearance. Typically these psoriatic plaques are discrete and well-demarcated, surrounded by normal looking skin.

In most cases plaques appear only in a limited area of the body, such as the extensor surfaces of limbs, in particular elbows and knees, and the trunk, in particular the sacral area. However, they are not uncommon in the nails, scalp or intertriginous areas in body folds and genitalia.

Most commonly the clinical course is chronic, with a tendency for fluctuations In disease severity which may be associated to external factors such as seasonal changes or internal factors such as stress, although patients can sometimes experience periods with no or very low disease activity.

No curative treatment for psoriasis is currently available. Mild to moderate degrees of the disease with involvement of only a limited body surface area are treated mainly with topical formulations containing corticosteroids, vitamin D analogues, salicylic acid and tar derivatives, alone or in combination. The market leading product is the combination of calcipotriol with bethametasone diproplonate, marketed under the tradename Dalvobet. Patients who initially respond well to one of these treatments may find over time that the treatment response decreases.

In the case of extensive psoriatic lesions retinoids can be used systemically. However, only one topical preparation containing a retinoid is commercially available: Zorac ®, a 0.05 % or 0.1 % tazarotene cream or gel.

There is thus a need for new topical treatments for the management of psoriasis having better efficacy than the regimens currently used as first line and also increasing the therapeutic options for difficult or refractory cases.

Combining multiple topical medications is a common approach to psoriasis therapy. An obvious advantage of combining corticosteroids and retinoids is the potential ability of corticosteroids to improve the efficacy of retinoid therapy and to reduce the incidence of retinoid-induced skin irritation.

Several clinical trials have established the benefit of using a topical corticosteroid in a concomitant treatment with tazarotene gel in the treatment on plaque psoriasis (Green et Sadoff, Journal of Cutaneous Medicine and Surgery, 2002, 6 (2), 95-102). The study reported in this article describes the application of different corticosteroids in the morning and tazarotene gel In the evening. Dhawan et al. describe a study with 10 patients having localized plaque-type psoriasis, treated with betamethasone valerate foam in the morning and topical tazarotene cream in the evening (Journal of Drugs in Dermatology, 2005. 4 (2), 228-230). More recently, van de Kerkhof compares the combination of tazarotene with various topical corticosteroids with tazarotene monotherapy and shows the superiority of the combination treatment (Dermatologic Therapy, 2006,19, 252-263).

In addition, some patent applications describe topical combinations of tazarotene with corticosteroids (CN1 478478, CN1528393, WO98/36753 or WOQ3/030896). However, until now there is no approved combination of tazarotene or any other retinoid with a corticosteroid for the topical treatment of psoriasis. This Is mainly due to the fact that obtaining stable formulations of two active ingredients with so different physicochemical properties is very difficult.

Bexarotene is a retinoid which is commercialized as Targretin® Gel in the US for the treatment of cutaneous lesions of chronic T cell lymphoma. It is a retinoid X receptor (RXR)-specific ligand, which binds to RXR (RXRα and RXRγ), but not to retinoic acid receptor (RAR).

Preliminary data from an open study on its use in psoriasis have been published by Breneman *et al.* (Breneman D. Sheth P, Berger V, Naini V, Stevens V J Drugs Dermatol. Phase II clinical trial of bexarotene gel 1% in psoriasis. 2007 May;6(5):501-6), who reported some initial improvement in psoriasis patients after up to 24 weeks treatment.

Surprisingly, the experimental data provided in this application show that the combination of corticosteroids with bexarotene produces anti-psoriatic effect which is larger than the effect of combining corticosteroids with calcipotriol (as in Daivobet ®, the reference commercial product) or with other retinoids currently used In monotherapy or in concomitant therapy like tazarotene. Besides, the topical pharmaceutical compositions of the invention are physically and chemically stable, easy to apply, and well accepted to patients. They are particularly suitable for application once daily.

### SUMMARY OF THE INVENTION

New topical pharmaceutical compositions have been developed comprising:
a) bexarotene,
b) a corticosteroid or any pharmaceutically acceptable salt thereof, and
c) a carrier or vehicle.

The Invention further relates to the use of a composition as defined above as defined above for use in the treatment or prevention of skin disorders.

The invention also relates to the use of a composition as defined above for the manufacture of a medicament for the treatment or prevention of skin disorders.

The invention further relates to a method for treating a subject afflicted with a skin disorder which comprises applying to the affected area of skin an effective amount of a topical pharmaceutical composition as defined above.

### DETAILED DESCRIPTION OF THE INVENTION

The Invention relates to a topical pharmaceutical composition comprising:
a) bexarotene,
b) at least a corticosteroid or any pharmaceutically acceptable salt or ester thereof, and
c) a carrier or vehicle.

According to the invention, the corticosteroid is selected from clobetasol, betamethasone, mometasone, prednicarbate, methylprednisolone, triamcinolone, halobetasol, halcinonide, desoximetasons, fluocinonide, hydrocortisone, prednisolone, fluocortolone, chlorocortolone, fluocinolone, diflucortolone, desonide, dexamethasone, alclomethasone and desoximethasone, or any pharmaceutically acceptable salt or ester thereof, or mixtures thereof.

Preferably, the corticosteroid is selected from clobetasol 17-propionate, betamethasone dipropionate, betamethasone valerate, mometasone furoate, prednicarbate, methylprednisolone aceponate, triamcinolone acetonide, halobetasol propionate, halcinonide, desoximatesone, fluocinonide, hydrocortisone, hydrocortisone 17-valerate, hydrocortisone acetate, prednisolone, fluocortolone pivalate, chlorocortolone plvalate, fluocinolone acetonide, diflucortolone valerate, desonide, dexamethasone and esters thereof, alclomethasone dipropionate and desoximethasone, or mixtures thereof.

In a preferred embodiment, the corticosteroid is selected from clobetasol 17-propionate, betamethasone dipropionate, betamethasone valerate, mometasone furoate, prednicarbate, methylprednisolone aceponate and triamcinolone acetonide, or mixtures thereof.

In a particularly preferred embodiment, the corticosteroid is betamethasone or any pharmaceutically acceptable salt thereof, such as betamethasone dipropionate, betamethasone sodium phosphate and betamethasone valerate; preferably betamethasone is in the form of betamethasone dipropionate.

Typically, the corticosteroid concentration is in the range of 0.01 wt. % to 0.3 wt. %, preferably in the range of 0.05 wt. % to 0.1 wt. %, based on the total weight of the composition.

According to one embodiment of the invention, in the composition according to the Invention the betamethasone is in the form of betamethasone dipropionate.

Typically, the betamethasone dipropionate concentration is in the range of 0.01 wt. % to 0.3 wt. %, preferably in the range of 0.05 wt. % to 0.1 wt. %, based on the total weight of the composition.

Typically, the bexarotene concentration is in the range of 0.1 wt.% to 2.0 wt.%, preferably in the range of 0.25 wt.% to 1 wt.%, more preferably 1 wt.%, based on the total weight of the composition.

Typically, the carrier or vehicle is selected from selected from water, petroleum hydrocarbons, fatty acids, fatty acid esters, fatty alcohols, fatty alcohol ethers, fatty alcohol esters, C2-C8 linear or branched, saturated or unsaturated alcohols, polyols, aromatic alcohols, alkyleneglycol ethers, alkyleneglycol esters, natural waxes and silicones or mixtures thereof.

Preferably, the carrier or vehicle is selected from fatty acids, fatty acid esters, fatty alcohols, fatty alcohol ethers, fatty alcohol esters, C₂-C₈ linear or branched, saturated or insaturated alcohols, polyols, aromatic alcohols, alkyleneglycol ethers, alkyleneglycol esters and natural or mixtures thereof.

The topical pharmaceutical compositions according to the invention may optionally further comprise other well-known pharmaceutically and/or cosmetically acceptable additives, such as, e.g. anti-irritants, antioxidants, buffering agents (pH adjusting agents), chelating agents, emollients, penetration enhancing agents, preservative agents, solubilizing agents, thickening agents, wetting agents, and the like, or mixtures thereof.

One embodiment of the invention relates to a topical pharmaceutical composition as defined above for use in the treatment or prevention of skin disorders.

Another embodiment of the invention relates to the use of a topical pharmaceutical composition as defined above for the manufacture of a medicament for the treatment or prevention of skin disorders.

A further embodiment of the invention relates to a method for treating a subject afflicted with a skin disorder which comprises applying to the affected area of skin of said subject an effective amount of a composition as defined above.

The method of using the topical pharmaceutical composition of the invention is by applying it to completely cover the affected area, forming an occlusive barrier. The usual frequency of application is once daily, although adequate maintenance therapy for some patients may be achieved with less frequent application.

As used herein, the term skin disorder refers to disorders of the skin with inflammation and/or infiltration patterns, Examples of these disorders are psoriasis, atopic dermatitis (atopic eczema), contact dermatitis, seborrheic dermatitis, xerotic eczema, scalp eczema, hand eczema, dyshidrosis, discoid eczema, venous eczema, dermatitis herpetiformis, neurodermatitis, autoeczematization, acne, rosacea and cutaneous T-cell lymphomas (CTLC) such as mycosis fungoides (MF) and Sézare syndrome (SS).

Preferably skin disorder refers to psoriasis, atopic dermatits (atopic eczema), hand eczema, and cutaneous T-cell lymphomas; more preferably skin disorder refers to psoriasis.

As used herein, the term corticosteroid refers to active pharmaceutical ingredients that closely resemble cortisol, a hormone produced in the adrenal cortex.

In general, corticosteroids are grouped into four classes, based on chemical structure:
- Group A - Hydrocortlsone type: hydrocortisone, hydrocortisone acetate, cortisone acetate, tixocortol pivalate, prednisolone, methylprednisolone, and prednisone
- Group B - Acetonides: triamcinolone acetonide, triamcinolone alcohol, mometasone, amcinonide, budesonide, desonide, fluocinonide, fluocinolone acetonide, and halcinonide.
- Group C - Betamethasone type: betamethasone, betamethasone sodium phosphate, dexamethasone, dexamethasone sodium phosphate, and fluocortolone.
- Group D - Esters (halogenated and labelled prodrug esters)

As used herein, the term "carrier" or "vehicle" refers to carrier material suitable for dermal drug administration and include any such material known in the art, e.g. any liquid, gel, solvent, liquid diluent, solubilizer or the like, which does not interact with other components of the composition in a deleterious manner. Examples of suitable carriers other than water can be found at Martindale - The complete drug reference, 32nd edition, 1999.

Suitable petroleum hydrocarbons, i.e. mineral oils, paraffins and waxes from petroleum according to the present invention are: hard paraffin, liquid paraffin (Liquid Petrolatum or Paraffinum Liquidum), light liquid paraffin (Light Liquid Petrolatum or Paraffinum Perliquidium), white soft paraffin (White Petrolatum), yellow soft paraffin (Yellow Petrolatum), macrocrystalline paraffin waxes (which are mixtures which consist mainly of saturated C₁₈-C₃₀ hydrocarbons and smaller amounts of iso-alkanes and cycloalkanes with a molecular weight comprised between 250 and 450 g/mol and, although they are solids at room temperature, they have low melting points, usually comprised between 40°C and 60°C), microcrystalline paraffines waxes (which consist of C₄₀-C₅₅ compounds which contain, in addition to normal hydrocarbons, large amounts of iso-alkanes and naphtenes with long alkyl side-chains, the iso-alkanes forming microcrystals, the microcrystalline paraffines waxes having mean molecular weights comprised between 500 and 800 g/mol, being solids at room temperature, and having melting points comprised between 60°C and 90°C), or mixtures thereof. Preferred petroleum hydrocarbons are hard paraffin, liquid paraffin, light liquid paraffin, white soft paraffin or mixture thereof, being particularly preferred liquid paraffin, white soft paraffin or mixtures thereof.

Suitable fatty acids according to the present invention are C₈-C₂₄ carboxylic acids, saturated or unsaturated, purified or synthetic, from vegetable and animal fats and oils, such as 2-ethylhexanoic acid, arachidic acid, arachidonic acid, behenic acid, capric acid, caproic acid, caprylic acid, castor oil acid, coconut acid, corn acid, cottonseed acid, elaidic acid, erucic acid, gadoleic acid, isostearic acid, lauric acid, linoleic acid, linolenic acid, linseed acid, myristic acid, oleic acid, olein, olive acid, olive pomace, palm acid, palm kernel acid, palmitic acid (cetylic acid), palmitoleic acid, peanut acid, pelargonic acid, petroselinic acid, rapeseed acid, rice bran acid, ricinoleic acid, safflower acid, soy acid, stearic acid, sunflower seed acid, tall oil acid, tallow acid, undecanoic acid, undecylenic acid, wheat germ acid or mixtures thereof.

Fatty acid esters as used herein represent the covalent compounds formed between the fatty acids as described above and any suitable alcohol. Suitable fatty acid esters according to the present invention are preferably selected from (i) fats and oils, (ii) alkyl fatty esters, (iii) alkoxylated fatty acid esters and (iv) sorbitan fatty acid esters- (i) Fats and oils are the glyceryl esters of fatty acids (triglycerides) normally found in animal and plant tissues, Including those which have been hydrogenated to reduce or eliminate unsaturation. Also included are synthetically-prepared esters of glycerin and fatty acids (mono-, di-, and triglycerides). The fatty acids esterifying the different positions of glycerin can be different, giving rise to a large amount of possible combinations, including positional combinations. The position of the different fatty acids in natural triglycerides is not random, but rather It depends on the origin of the fat. The triglycerides more simple are those constituted by a sole fatty acid. Glyceryl esters of fatty acids can be advantageously chosen, for example, from the group consisting of synthetic, semi-synthetic and natural oils, as for example, animal fats and oils such as cow tallow, pig lard, bone oil, aquatic animal fats and oils (fish, such as herring, cod or sardine; cetaceans; etc.); and vegetable fats and oils such as avocado oil, almond oil, hazelnut oil, babassu palm oil, borage oil, peanut oil, canola oil, hemp oil, milk thistle oil, safflower oil, chufa oil, coconut oil, rapeseed oil, black cumin oil, wheat germ oil, sunflower oil, linseed oil, macadamia nut oil, corn oil, walnut oil, olive oil and Its byproducts such as olive pomace oil, palm oil and its fractions such as palm olein and palm stearin, evening primrose oil, rosehip oil, castor oil, rice bran oil, apricot kernel oil, cottonseed oil, pumpkinseed oil, palm kernel oil and its fractions such as palm kernel olein and palm kernel stearin, grape seed oil, sesame oil, soy oil, cocoa butter, shea butter and the like. Other examples of glyceryl esters are glyceryl monobehenate, glyceryl dibehenate, glyceryl monooleate, glyceryl dioleate, glyceryl monostearate, glyceryl distearate, glyceryl monopalmitosteareate and glyceryl dipalmitosteareate. (ii) Alkyl fatty esters represent esters of fatty acids as described above and linear or branched, saturated or unsaturated C₁-C₃₀ alcohols, ethylene glycol or propylene glycol. These fatty acid esters can be advantageously selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl laurate, 2-ethylhexyl palmitate. 2-ethylhexyl cocoate, 2-hexyldecyl stearate, 2- ethylhexyl isostearate, 2-octyldodecyl palmitate, cetyl palmitate, oleyl oleate, oleyl erucate, erucyl oleate, erucyl erucate, propyleneglycol monocaprylate, propyleneglycol dicaprylate, propyleneglycol monopalmitostearate, propyleneglycol monostearate, propyleneglycol alginate, ethyleneglycol monopalmitostearate, ethyleneglycol monostearate, as well as synthetic, semisynthetic and natural mixtures of such esters, such as jojoba oil (a natural mixture of esters of monounsaturated monocarboxylic acids with a C₁₈-C₂₄ chain with also monounsaturated monoalcohols and with a long C₁₀-C₂₄ chain). (iii) Alkoxylated fatty acid esters are formed when a fatty acid is reacted with an alkylene oxide or with a preformed polymeric ether. The resulting product may be a monoester or a diester or a mixture of the two, depending on the reaction conditions. Typical representatives include PEG-6 isosteareate, PEG-2 stearate, PEG-4 stearate, PEG-8 stearate, PEG-12 stearate, PEG-20 stearate, PEG-40 stearate, PEG-50 stearate, PEG-100 stearate and PPG-17 dioleate. (iv) Sorbitan fatty acid esters are the reaction product of the esterification of sorbitan an one or more fatty acids as defined above. Examples of suitable sorbitan fatty acid esters include sorbitan monolaurate, sorbitan monooleate, sorbitan monopalmltate, sorbitan monostearate, sorbitan sesquioleate, sorbitan trioleate and sorbitan tristearate, and polysorbates such as polysorbate 20, polysorbate 40, polysorbate 60, polysorbate 65, polysorbate 80 or polysorbate 85.

Suitable fatty alcohols according to the present invention are C₆-C₂₄ alcohols from vegetable and animal fats and oils, such as 2-octyldodecanol, 2-ethylhexanoyl alcohol, arachidyl alcohol, behenyl alcohol, caprylic alcohol, caproyl alcohol, capric alcohol, castor oil alcohol, ceterayl alcohol, palmityl (cetyl) alcohol, coconut alcohol, cotton alcohol, decyl alcohol, elaidyl alcohol, erucyl alcohol, gadoleyl alcohol, isostearyl alcohol, lauryl alcohol, linoleyl alcohol, linseed alcohol, myristyl alcohol, olein alcohol, olive pomace alcohol, oleyl alcohol, olive alcohol, palm alcohol, palm kernel alcohol, palmitoyl alcohol, petroselinic alcohol, rapeseed alcohol, ricinoleyl alcohol, safflower alcohol, soy alcohol, stearyl alcohol, sunflower alcohol, tall oil alcohol, tallow alcohol, tridecyl alcohol, or technical grade mixtures thereof such as cetostearyl alcohol.

Suitable fatty alcohol ethers according to the present invention are ethers formed from the reaction of a fatty alcohol as defined above with an alkylene oxide, generally ethlyene oxide or propylene oxide. Fatty alcohols ethers can be advantageously selected from the group consisting of ceteth-20, isosteareth-10, myreth-10, laureth-16, oleth-16, polyoxyl 6 cetostearyl ether, polyoxyl 20 catostearyl ether, polyoxyl 25 cetostearyl ether, polyoxyl 2 cetyl ether, polyoxyl 10 cetyl ether, polyoxyl 20 cetyl ether, polyoxyl 4 lauryl ether, polyoxyl 9 lauryl ether, polyoxyl 23 lauryl ether, polyoxyl 2 oleyl ether, polyoxyl 10 oleyl ether, polyoxyl 20 oleyl ether, polyoxyl 2 stearyl ether, polyoxyl 10 stearyl ether, polyoxyl 21 stearyl ether or polyoxyl 100 stearyl ether.

Suitable fatty alcohol esters are the product of the reaction of a fatty alcohol as defined above and a linear of branched, saturated or unsaturated C₁-C₅ carboxylic acid. Examples of fatty alcohol esters are lauryl acetate, myristyl acetate, cetyl acetate, stearyl acetate and stearyl propionate.

Suitable aromatic alcohols according to the present invention are preferably selected from (i) arylalkanols, (ii) aryloxyalkanols (glycol monoaryl ethers) and (iii) oligoalkanol aryl ethers. The (i) arylalkanols used according to the Invention have the formula Ar-(CHR)ₙ-OH where R independently represents H or C₁-C₆ alkyl, with n being an integer, and preferably 1 to 10, more preferably 1 to 6, and In particular 1, 2, 3 or 4. The group Ar can be a substituted or unsubstituted aryl group, for example phenyl or naphtyl. Example arylalkanols are benzyl alcohol, 3-phenylpropan-1-ol, phenethyl alcohol, veratryl alcohol (3,4-dimethoxyphenylmethyl alcohol) and 2-methyl-1-phenyl-2-propanol. The (ii) aryloxyalkanols used according to the invention have the formula Ar-O-(CHR)ₙ-OH where R independently represents H or C₁-C₆ alkyl, with n being an integer, and preferably 2 to 10, more preferably 2 to 6, and in particular 2 or 3. The group Ar can be a substituted or unsubstituted aryl group, for example phenyl or naphtyl. Example arlyoxyalkanols used according to the invention are phenoxyethanol, 1-phenoxypropan-2-ol, 2-phenoxylpropan-1-ol, 3-phenoxypropan-1-ol, or mixtures thereof. The (iii) oligoalkanol aryl ethers include, for example, phenoxy diethanol, triethanol and oligoethanol, and phenoxy dipropanol, tripropanol and oligopropanol.

Suitable polyols according to the present invention are preferably water-soluble polyols such as polyhydric alcohols with two or more hydroxyl groups in their molecule. Specific examples can Include ethylene glycol, propylene glycol, 1,3- butylene glycol, 1,4-butylene glycol, hexylene glycol, dipropylene glycol, glucose, fructose, galactose, mannose, ribose, erythrose, maltose, maltitose, maltotriose, sucrose, xylitol, sorbitol, threitol, erythritol, glycerol, polyglycerol and starch alcohols. Preferred polyols are ethylene glycol, propylene glycol, 1,3-butylene glycol, 1,4-butylene glycol, dipropylene glycol, hexylene glycol, glycerol, polyglycerol, and mixtures thereof.

Suitable alkyleneglycol esters are esters of ethylene glycol or propylene glycol with the C₆-C₂₄ fatty acids defined above. Alkyleneglycol esters can be advantageously selected from the group consisting of ethylene glycol monopalmitostearate, ethylene glycol monostearate, propylene glycol monocaprylate, propylene glycol diceprylate, propylene glycol dicaprylocaprate, propylene glycol monopalmitostearate, propylene glycol monostearate or propylene glycol alginate.

Suitable alkylene glycol ethers are polymers of ethylene oxide (polyethylene glycol monomethyl ether) or propylene oxide (polypropylene glycol monomethyl ether). Alkyleneglycol ethers can be advantageously selected from the group consisting of PEG 200, PEG 400, PEG 540, PEG 600, PEG 900, PEG 1000, PEG 1450, PEG 1540, PEG 2000, PEG 3000, PEG 3350, PEG 4000, PEG 4600, PEG 8000, PPG-9, PPG-10, PPG-17, PPG-20, PPG-26, PPG-30 or PPG-55.

Suitable natural waxes according to the present invention are the candelilla wax, carnauba wax, Japan wax, esparto wax, cork wax, guaruma wax, rice wax, sugar cane wax, ouricury wax, montan wax, beeswax, shellac wax, espermaceti, wool lanolin (wax), uropygial fat wax, ceresin waxes, peat waxes, ozokerite, as well as chemically modified waxes (hard waxes) for example, montan wax esters, waxes obtained by the Fischer-Tropsch process, hydrogenated jojoba waxes and synthetic waxes.

Silicones suitable according to the present invention are cyclic and/or linear silicones, which can be found as monomers generally characterized by structural elements such as: 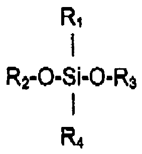 where the silicon atoms can be substituted by alkyl or aryl radicals equal or different, represented here generally by R₁-R₄ groups.

Linear silicones with siloxane units suitable according to the present invention are generally characterized by structural elements such as: 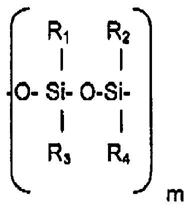 where the silicon atoms can be substituted by alkyl or aryl radicals equal or different, are represented here in general by R₁-R₄ groups (meaning the number of different radicals is not necessarily limited to 4), m can take values from 2 to 200.000.

Cyclic silicones suitable according to the present invention are generally characterized by structural elements such as: 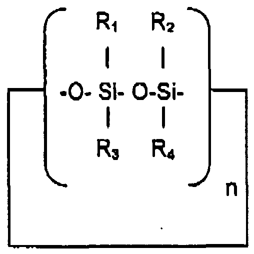 where the silicon atoms can be substituted by alkyl or aryl radicals equal or different, represented here generally by R₁-R₄ groups (meaning the number of different radicals is not necessarily limited to 4), n can take values of 3/2 to 20. Fractional values of n indicate that it may be odd numbers of siloxane groups present in the ring.

Specific examples include a cyclic methyl siloxane having the formula [(CH₃)₂SiO]ₓ in which x is 3-6, or short chain linear methyl siloxanes having the formula ((CH₃)₂SiO[(CH₃)₂SiO]_{y}Si(CH₃)₃ in which y is 0-5.

Some suitable cyclic methyl siloxanes are hexamethylcyclotrisiloxanes (D3), a solid with a boiling point of 134°C and the formula [(Me₂)SiO]₃; octamethylcyclotetrasiloxane (D4) with a bolling point of 176°C, a viscosity of 2.3 mm²/s, and the formula [(Me₂)SiO]₄; decamethylcyclopentasiloxane (D5) (cyclomethicone) with a boiling point of 210°C, a viscosity of 3.87 mm²/s_{,} and the formula [(Me₂)SiO]₅; and dodecamethylcyclohexasiloxane (D6) with a boiling point of 245°C, a viscosity of 6.62 mm²/s and the formula [(Me₂)siO]₆.

Some suitable short linear methyl siloxane are hexamethyldisiloxane (MM) with a boiling point of 100°C, viscosity of 0-65 mm²/s, and formula Me₃SiOMe₃; octamethyltrisiloxane (MDM) with a boiling point of 152°C, viscosity of 1.04 mm²/s, and formula MeₛSiOMe₂SiOSiMe₃; decamethyltetrasiloxane (MD2M) with a boiling point of 194°C, viscosity of 1.53 mm²/s, and formula Me₃SiO(MeSiO)₂SiMe₃; dodecamethylpentasiloxane (MD3M) with a boiling point of 229°C, viscosity of 2.06 mm²/s, and formula Me₃SiO(Me₂SiO)₃SiMe₃; tetradecamethylhexasiloxane (MD4M) with a boiling point of 245°C, viscosity of 2.63 mm²/s, and formula Me₃SiO(Me₂SiO)₄SiMe₃; and hexadecamethylheptasiloxane (MD5M) with a boiling point of 270°C, viscosity of 3.24 mm²/s, and formula Me₃SiO(Me₂SiO)₅SiMe₃.

Furthermore, long chain linear siloxanes such as phenyltrimethicone, bis(phenylpropyl)dimethicone, dimethicone, dimethiconol, cyclomethicone (octametilciclotetrasiloxane), hexamethylcyclotrisiloxane, poly(dimethylsiloxane), cetyldimothicone and behenoxy dimethicone are also included.

In addition, mixtures of cyclomethicone and isotridecyl isononanoate and of cyclomethicone and 2-ethylhexyl isostearate are also suitable silicones according to the invention.

The topical pharmaceutical composition according to the invention can be formulated in the form of a cream, a gel, an oleogel, an ointment, a paste, a suspension, a lotion, a foam, a spray, an aerosol or a solution, preferably in the form of a cream, an ointment or a lotion.

The Food and Drug Administration (FDA), Center for Drug Evaluation and Research (CDER) Data Standards Manual, Dosage Form (version 08) defines ointment as "a semisolid dosage form, usually containing less than 20% water and volatiles and more than 50% hydrocarbons, waxes, or polyols as the vehicle, which is generally for external application to the skin or mucous membranes".

The Food and Drug Administration (FDA), Center for Drug Evaluation and Research (CDER) Data Standards Manual, Dosage Form (version 08) defines cream as "an emulsion, semisolid dosage form, usually containing more than 20% water and volatiles and/or less than 50% hydrocarbons, waxes, or polyols as the vehicle, which is generally for external application to the skin or mucous membranes".

The topical pharmaceutical compositions according to the invention may optionally further comprise other well-known pharmaceutically and/or cosmetically acceptable additives, such as, e.g, anti-irritants, antioxidants, buffering agents (pH adjusting agents), chelating agents, emollients, penetration enhancing agents, preservative agents, solubilizing agents, thickening agents, wetting agents, and the like, or mixtures thereof.

Examples of suitable anti-irritants are aloe vera, chamomile, alpha-bisabolol, cola nitida extract, green tea extract, tea tree oil, licoric extract, batyl alcohol (α-octadecyl glyceryl ether), selachyl alcohol (α-9-octadecenyl glyceryl ether), chimyl alcohol (α-hexadecyl glyceryl ether), panthenol, allantoin, caffeine or other xanthines, glycyrrhizic acid and derivatives thereof, and mixtures thereof.

Antioxidants used can be any antioxidants which are suitable or customary for cosmetic and/or dermatological applications. Suitable antioxidants are advantageously selected from the group consisting of amino acids (for example glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) and sulphoximine compounds (e.g. buthionine sulphoximines, homocysteine sulphoximine, buthionine sulphones, penta-, hexa-, heptathionine sulphoximine) in very small tolerated doses (e.g. pmol to µmol/kg), also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), and coniferylbenzoate of benzoin, rutinic acid and derivatives thereof, ferulic acid and derivatives thereof, butylated hydroxytoluene, butylated hydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenium methionine), stilbenes and derivatives thereof (e.g. stilbene oxide, trans-stilbene oxide) and the derivatives (salts, esters, ethers, sugars, nucleotides, nucleosides, peptides and lipids) of said active ingredients which are suitable according to the invention.

Any pharmaceutically acceptable buffering agents to adjust the pH of the topical pharmaceutical compositions according to the invention to be within the acceptable range for topical administration, preferably In the range of 3.0 to 6.0, more preferably in the range of 3.5 to 5.0, can be used. For example the inclusion in the composition of a pharmaceutically acceptable acid such as acetic, citric, fumaric, phosphoric, hydrochloric, lactic or nitric acids or the like, or a mixture thereof. It will also be understood that certain compositions of the invention can have a pH in the desired range without inclusion of a pH adjusting agent specifically for that purpose. Typically, however, an acidic buffer system is present in the composition to achieve the desired pH. An acidic buffer system comprises an acidulant and a buffering agent. Suitable acidulants will be known to those of skill in the art and illustratively include acetic, citric, fumaric, hydrochloric, phosphoric, lactic and nitric acids and the like, and mixtures thereof. Suitable buffering agents will likewise be known to those of skill in the art and illustratively include potassium metaphosphate, potassium phosphate, sodium phosphate, sodium acetate, sodium citrate and the like, and mixtures thereof.

Suitable emollients, which can be used in the composition of the present invention include, for example, dodecane, squalane, cholesterol, isohexadecane, isononyl isononanoate, PPG ethers, petrolatum, lanolin, safflower oil, castor oil, coconut oil, cottonseed oil, palm kernel oil, palm oil, peanut oil, soybean oil, polyol carboxylic acid esters, derivatives thereof, and the like, and combinations thereof.

Examples of suitable penetration enhancing agents can Include, e.g., dimethylsulfoxide (DMSO), N-methyl pyrrolidine, dimethyl formamide (DMF), allantoin, urazole, N,N-dimethylacetamide (DMA), decylmethylsulfoxide, polyethylene glycol monolaurate, propylene glycol, propylene glycol monolaurate, glycerol monolaurate, lecithin, the 1-substituted azacycloheptan-2-ones, particularly 1-n-dodecylcyclazacycloheptan-2-one, alcohols, glycerin, hyaluronic acid, transcutol, and the like, and combinations thereof. Certain oil components (e.g., certain vegetable oils such as, e.g., safflower oil, cottonseed oil and corn oil) also can exhibit penetration enhancing properties.

Examples of suitable preservatives to prevent microbial contamination are alkylparabens, particularly methylparaben, propylparaben and butylparaben; sodium benzoate; butylated hydroxy toluene; butylated hydroxyanisole; ethylenediamine tetraacetic acid; chlorobutanol; benzyl alcohol; phenylethylalcohol; dehydroacetic acid; sorbic acid; potassium sorbate; benzalkonium chloride; benzethonium chloride; and mixtures thereof. The amount of preservative generally utilized will vary depending upon the preservative selected.

Examples of solubilizing agents are, for example, nonionic surfactants from at least one of the following groups: products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group; alkyl and/or alkenyl oligoglycosides containing 8 to 22 carbons in the alkyl group and ethoxylated analogs thereof; addition products of 1 to 15 moles of ethylene oxide with castor oil and/or hydrogenated castor oil; addition products of 15 to 60 moles of ethylene oxide with castor oil and/or hydrogenated castor oil; partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide; mixtures of alkoxylated glycerides and alkoxylated glycerine, partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide; mixed esters of pentaerythritol, fatty acids; citric acid and fatty alcohol and/or mixed esters of fatty acids containing 6 to 22 carbons, methyl glucose and polyols, preferably glycerol or polyglycerol; mono-, di- and trialkyl phosphates and mono-, di-and/or tri-PEG-alkyl phosphates and salts thereof; block copolymers, for example Polyethyleneglycol-30 Dipolyhydroxystearate; polymer emulsifiers; polyalkylene glycols and alkyl glyceryl ethers. Particularly preferred solubilizing agents are products of the addition of 1 to 30 moles of ethylene oxide and/or 0 to 5 moles of propylene oxide onto linear C₆-C₂₂ fatty alcohols such as lauryl, myristyl, cetyl (palmityl), stearyl, oleyl, and ricinoleyl alcohols, or technical grade mixtures thereof such as cetostearyl alcohol or palmitoleyl alcohol.

A thickening agent or viscosity-enhancing agent can be included to generally thicken the liquid pharmaceutical compositions. While any suitable thickening agent can be included in the compositions of the present invention, a preferred thickening agent, when used, includes one or more of acacia, alginic acid bentonite, carbomer, carboxymethylcellulose calcium or sodium, cetostearyl alcohol, methyl cellulose, ethylcellulose, glycerin, gelatin guar gum, hydroxyethylcellulose, hydroxypropylcellulose, hydroxypropylmethylcellulose, maltodextrin, polyvinyl alcohol, povidone, propylene carbonate, propylene glycol alginate, sodium alginate, sodium starch glycolate, starch tragacanth, and xanthan gum, and any combination thereof. More preferred thickening agents are glycerin, hydroxypropylmethylcellulose, and xanthan gum, and any combination thereof.

Examples of wetting agents (chemical substances that increase the spreading and penetrating properties of a liquid by lowering its surface tension) include one or more cationic surfactants, such as benzalkonium chloride; non-ionic surfactants such as polyoxyethylene and polyoxypropylene block copolymers; polyoxyethylene fatty acid glycerides and oils (such as polyoxyethylene (6) caprylic/capric mono- and diglycerides), polyoxyethylene (40) hydrogenated castor oil; polyoxyethylene sorbitan esters, such as polysorbate 20 and polysorbate 80; propylene glycol fatty acid esters, such as propylene glycol laureate; glyceryl fatty acid esters, such as glyceryl monostearate; sorbitan esters, such as sorbitan monolaurate, sorbitan monooleate, sorbitan monopalmitate and sorbitan monostearate; glyceryl fatty acid esters, for example glyceryl monostearate; anionic surfactants such as sodium lauryl sulphate, sodium lauryl ether sulphate; or fatty acids and salts thereof, such as oleic acid, sodium oleate and triethanolemine oleate.

The viscosity of the topical pharmaceutical composition of the invention will depend on the form of the composition. For instance, in the case of a cream, the viscosity is typically in the range of 2,000 to 15,000 mPa.s, preferably in the range of 2,500 to 10,000 mPa.s, more preferably in the range of 3,000 to 7,000 mPa.s measured at 20°C using a DIN-Rotations Rheometer (Paar Physica); Measuring System Z 3 DIN; D= 57 1/s.

In the case of a gel, the viscosity is typically in the range of 300 to 1,500 mPa.s, preferably in the range of 500 to 1,200 mPa.s, more preferably in the range of 600 to 900 mPa.s measured at 20°C using a DIN-Rotations Rheometer (Paar Physica); Measuring System Z 3 DIN ; D= 57.2/s.

The following examples are given in order to provide a person skilled in the art with a sufficiently clear and complete explanation of the present invention, but should not be considered as limiting of the essential aspects of its subject, as set out in the preceding portions of this description.

### EXAMPLES

### Examples 1-6

Compositions according to the invention were prepared as indicated in Table 1 (wt.% based on the total weight of the composition)

**Table 1**

| **Ingredients** | **wt.%** | | | | | |
|---|---|---|---|---|---|---|
| | **Ex. 1** | **Ex. 2** | **Ex. 3** | **Ex. 4** | **Ex. 5** | **Ex. 6** |
| Bexarotene | 0.5 | 1.00 | 1.00 | 0.5 | 0.25 | 1.00 |
| Betamethasone dipropionate | 0.10 | 0.10 | 0.05 | 0.05 | 0.05 | 0.10 |
| Octyldodecanol | 35 | 35 | 35 | 35 | 35 | 60 |
| White soft paraffin | 61.35 | 60.85 | 60.90 | 61.40 | 61.65 | 35.85 |
| Propyleneglycolpalmitostearate | 3.00 | 3.00 | 3.00 | 3.00 | 3.00 | 3.00 |
| Citrate buffer, 8 mM | 0.05 | 0.05 | 0.05 | 0.05 | 0.05 | 0.05 |

Said compositions were prepared in the following manner:
1) Octyldodecanol was added to a stainless steel container and heated to 80°C while stirring. Bexarotene was added and dissolved while stirring to obtain a clear solution.
2) White soft paraffin and propylene glycol monopalmitostearate were added to a stainless steel container. The ingredients were heated to 60°C and melted while stirring. The melted mixture of lipophilic compounds was homogenous and clear.
3) Betamethasone dipropionate was transferred to the melted mixture of lipophilic compounds and suspended (distributed thoroughly) while homogenizing.
4) The bexarotene solution was transferred to the batch while stirring.
5) Anhydrous citric acid and sodium citrate were dissolved in the purified water and this solution was transferred to the batch while homogenizing.
6) The batch was cooled down to 30°C while stirring.
7) The batch was then filled directly into the glass bottles.

### Examples 7-9

Compositions according to the invention were prepared as Indicated in Table 2 (wt.% based on the total weight of the composition).

**Table 2**

| **Ingredients** | **wt.%** | | |
|---|---|---|---|
| | **Ex. 7** | **Ex. 8** | **Ex. 9** |
| Bexarotene | 0.25 | 0.5 | 1.00 |
| Betamethasone dipropionate | 0.05 | 0.05 | 0.10 |
| Paraffinum liquidum | 5.00 | 5.00 | 5.00 |
| White soft paraffin | 91.65 | 91.40 | 90.85 |
| Propyleneglycolpalmitostearate | 3.00 | 3.00 | 3.00 |
| Citrate buffer, 8 mM | 0.05 | 0.05 | 0.05 |

Said compositions were prepared in the following manner:
1) Liquid paraffin, white soft paraffin and propylene glycol monopalmitostearate were added to a stainless steel container. The ingredients were heated to 60°C and melted while stirring. The melted mixture of lipophilic compounds was homogenous and clear.
2) Bexarotene and betamethasone dipropionate were transferred to the melted mixture of lipophilic compounds and suspended (distributed thoroughly) while homogenizing.
3) Anhydrous citric acid and sodium citrate were dissolved in the purified water, and this solution was transferred to the batch while homogenizing.
4) The batch was cooled down to 30°C while stirring.
5) The batch was then be filled directly into the glass bottles.

### Example 10 - Efficacy

A psoriasis bio-assay for topical corticosteroid activity - psoriasis plaque test (assay described by Dumas and Scholtz, Acta Dermatovener (Stockh), 52, 43-48 (1972)) was also used to determine the efficacy of the following compositions:
a) Composition of Ex. 2,
b) Daivobet ® ointment, which contains calcipotriol (50 microgram/g) and betamethasone dipropionate (0.5 milligram/g), and
c) Tazarotene (0.2 wt.%) + Betamethasone dipropionate (0.1 wt.%) ointment.

Study population: Twenty-two male or female subjects were enrolled with chronic plaque psoriasis on trunk and/or extremities.

Test performance: Semi-occlusive application of the tests compositions for 10 days over 14 day time frame, with final reading after 1 day later.

The efficacy was assessed by reduction of the area under the curve (AUC) of the width of the echo-lucent band (ELB) located at the dermo-epidermal junction (representing the combination of acanthotic epidermal thickening and inflammation in psoriasis) as measured by 20 MHz ultrasound at visits 1, 4, 8 and 11. The results are indicated in Table 3.

**Table 3**

| **Composition** | **% of reduction In AUC of the ELB** |
|---|---|
| Ex. 2 | 94.9 |
| Daivobet ointment | 85.8 |
| Tazarotene + Betamethasone dipropionate ointment* | 70.9 |
| Base line (White soft paraffin) | 49.0 |

| | |
|---|---|
| * Different concentrations of tazarotene (from 0.05 to 0.2 wt.%) and betamethasone dipropionate (from 0.05 to 0.1 wt.%) were tested in the combination. In all cases, around 70 % of reduction in AUC of the ELB was observed. The results for the highest dose of both compounds is shown in Table 3. | |

These results demonstrate a better efficacy of the composition of the invention when compared with current benchmarks products for topical psoriasis treatment such as Daivobet or with the combination of tazarotene and a corticosteroid such as betamethasone.

Modifications, which do not affect, alter, change or modify the essential aspects of the pharmaceutical compositions described, are included within the scope of the present invention.

## Claims

1. A topical pharmaceutical composition comprising:
a) bexarotene,
b) at least a corticosteroid or any pharmaceutically acceptable salt or ester thereof, and
c) a carrier or vehicle.

2. A composition according to claim 1, wherein the corticosteroid is selected from clobetasol, betamethasone, mometasone, prednicarbate, methylprodnisolone, triamcinolone, halobetasol, halcinonide, desoximetasone, fluocinonide, hydrocortisone, prednisolone, fluocortolone, chlorocortolone, fluocinolone, diflucortolone, desonide, dexamethasone, alclomethasone and desoximethasone, or any pharmaceutically acceptable salt or ester thereof, or mixtures thereof.

3. A composition according to claim 1 or claim 2, wherein the corticosteroid is selected from clobetasol 17-propionate, betamethasone diproplonate, betamethasone valerate, mometasone furoate, prednicarbate, methylprednisolone aceponate, triamcinolone acetonide, halobetasol propionate, halcinonide, desoximetasone, fluocinonide, hydrocortisone, hydrocortisone 17-valerate, hydrocortisone acetate, prednisolone, fluocortolone pivalate, chlorocortolone pivalate, fluocinolone acetonide, diflucortolone valerate, desonide, dexamethasone and esters thereof, alclomethasone dipropionate and desoximethasone, or mixtures thereof.

4. A composition according to any one of the preceding claims wherein the corticosteroid is selected from clobetasol 17-propionate, betamethasone dipropionate, betamethasone valerate, mometasone furoate, prednicarbate, methylprednisolone aceponate and triamcinolone acetonide, or mixtures thereof.

5. A composition according to any one of the preceding claims, wherein the corticosteroid is betamethasone or any pharmaceutically acceptable salt thereof.

6. A composition according to claim 5, wherein the betamethasone is in the form of betamethasone dipropionate.

7. A composition according to any one of the preceding claims, wherein the corticosteroid concentration is in the range of 0.01 wt. % to 0.3 wt. %, based on the total weight of the composition.

8. A composition according to any one of the preceding claims, wherein the bexarotene concentration is in the range of 0.1 wt. % to 2.0 wt. % based on the total weight of the composition.

9. A composition according to any one of the preceding claims, wherein the carrier or vehicle is selected from from water, petroleum hydrocarbons, fatty acids, fatty acid esters, fatty alcohols, fatty alcohol ethers, fatty alcohol esters, C₂-C₈ linear or branched, saturated or insaturated alcohols, polyols, aromatic alcohols, alkyleneglycol ethers, alkyleneglycol esters, natural waxes, silicones or mixtures thereof.

10. A composition according to claim 9, wherein the carrier or vehicle is selected from fatty acids, fatty acid esters, fatty alcohols, fatty alcohol ethers, fatty alcohol esters, C₂-C₈ linear or branched, saturated or insaturated alcohols, polyols, aromatic alcohols, alkyleneglycol ethers, alkyleneglycol esters and natural or mixtures thereof.

11. A composition according to any one of the preceding claims, wherein the composition further comprises anti-irritants agents, antioxidants agents, buffering agents, chelating agents, amollients, penetration enhancing agents, preservative agents, solubilizing agents, thickening agents, wetting agents or mixtures thereof.

12. A composition as defined in any one of claims 1 to 11, for use in the treatment or prevention of skin disorders.

13. Use of a composition as defined in any one of claims 1 to 11, for the manufacture of a medicament for the treatment or prevention of skin disorders.

14. Method for treating a subject afflicted with a skin disorder which comprises applying to the affected area of skin of said subject an effective amount of a composition as defined in any one of claims 1 to 11.
